# EUROPÄISCHE PATENTSCHRIFT

(11) **EP 0 303 212 B1**
(45) Veröffentlichungstag und Bekanntmachung des Hinweises auf die Patenterteilung: **03.02.1993**
(21) Anmeldenummer: 88112858.1
(22) Anmeldetag: 06.08.1988
(51) Int. Cl.: A61L 9/01

(54) **Desodorantien, ihre Herstellung und Verwendung**
Deodorizer, preparation and use thereof
Désodorisant, sa préparation et son utilisation

(30) Priorität: 11.08.1987 DE 3726636
(43) Veröffentlichungstag der Anmeldung: 15.02.1989
(73) Patentinhaber: GRILLO-WERKE AG, D-47169 Duisburg (DE)
(72) Erfinder: Rohe, Dieter, Dr., D-4220 Dinslaken (DE); Bubel, Peter, D-4100 Duisburg 11 (DE); Driemel, Klaus, D-4100 Duisburg 25 (DE)
(74) Vertreter: Werner, Hans-Karsten, Dr.Dipl.-Chem.

(56) Entgegenhaltungen:
- EP-A- 0 046 970
- EP-A- 0 071 025
- US-A- 4 172 123
- CHEMICAL ABSTRACTS, Band 86, 1977, Seite 345, Zusammenfassung Nr. 60402t, Columbus, Ohio, US; N. LOWICKI et al.: "Deodorizing with grillozin", & KOSMETIKA (ZURICH) 1973, 1(2), 47-51
- CHEMICAL ABSTRACTS, Band 93, 1980, Seite 306, Zusammenfassung Nr. 191202v, Columbus, Ohio, US; N. LOWICKI et al.: "Deodorizing waste gases by chemical absorption of odorous substances in the wet-washing process", & DECHEMA-MONOGR. 1979 (Pub. 1980). 86(1743-1774)
- CHEMIKER ZEITUNG, Band 110, Nr. 2, Februar 1986, Seiten 63-68, Heidelberg, DE; H.-W. HENNIG et al.: "Bindung von Geruchsemissionen bei Abwasser-, Abluft- und Kompostierungsanlagen, Teil I - Einführung in die Problematik"

## Beschreibung

Gegenstand der vorliegenden Erfindung sind Desodorantien, insbesondere solche auf Basis von Zink-Ricinoleat mit Zinkverbindungen von mehrfach hydroxylierten höheren Fettsauren, Oxaminen und Harzsauren, Lösungmitteln aus der Gruppe der Alkohole, gegebenenfalls unter Zusatz von Wasser, Triethanolamin und organischen Säuren sowie Lösungsvermittlern, ihre Herstellung und ihre Verwendung im technischen Bereich, insbesondere zur Geruchsbeseitigung in Kläranlagen, Deponien, Haushaltungen und Krankenhäusern, in der Landwirtschaft, Tierzucht, Tierverwertung sowie in Fleisch- und Fischbetrieben.

Desodorantien dieser Art sind bekannt und werden bisher vor allem in der Kosmetik verwendet. So sind derartige Produkte als Aerosole für Raumspray, Deospray, Deo-Antitranspirantspray und Deo-Fußspray bekannt sowie Deo-Shampoos, Deo-Duschbäder, Deo-Stifte, Deo-Cremes, Deo-Seifen und Deo-Roll-ons.

Die wichtigste Komponente in diesen Desodorantien ist Zink-Ricinoleat, dessen Wirkung durch Zinkverbindungen von mehrfach hydroxylierten höheren Fettsäuren, Oxaminen und Harzsäuren synergistisch gesteigert werden kann. Ein derartiges Zink-Ricinoleat mit synergistischen Zusätzen wird von der Anmelderin seit vielen Jahren unter der Bezeichnung GRILLOCIN® HY-77 hergestellt und vertrieben. In den Rezepturen für die kosmetischen Produkte sind zusätzlich Lösungsmittel und Lösungsvermittler vorhanden. Als Lösungsmittel werden meistens ein- und mehrwertige Alkohole, gegebenenfalls unter Zusatz von etwas Wasser, verwendet, und als Lösungsvermittler haben sich Natriumdiglycol-Ricinosulfosuccinat bzw. Dinatriumpolyethylenglycol-Ricinosuccinat bewährt.

Diese Lösungsvermittler werden von der Anmelderin unter den Bezeichnungen GRILLOSOL® 8 C und GRILLOSOL^{®} 8 C 12 hergestellt und angeboten. Die Herstellung derartiger Lösungsvermittler ist in dem Europa-Patent 46 970 beschrieben.

EP-A-71025 offenbart kosmetische Rezepturen auf der Basis von Öl-/Wasser-Suspensionen enthaltend das Zinksabz der hydrolysierten Tricarbonsäure aus Diels-Alder-Addukten von Maleinsäureanhydrid an Undecylensäure (bakterizider und fungizider Wirkstoff) die jedoch aufgrund ihrer Beschaffenheit (Öl in Wasser-Suspension) nicht stabil sind.

Die Erfindung hat sich zunächst die Aufgabe gestellt, Desodorantien zu entwickeln auf Basis von Zink-Ricinoleat mit Zinkverbindungen von mehrfach hydroxylierten höheren Fettsäuren, Oxaminen und Harzsäuren, die auch in größerem Maßstab und insbesondere im technischen Bereich eingesetzt werden können. Man weiß, daß die bekannten Desodorantien in der Lage sind, insbesondere Mercaptane, Thioether, Amine und Aldehyde zu binden. Nicht gebunden werden hingegen Ketone, Alkohole und Ester. Die desodorierende Wirkung ist im einzelnen noch nicht vollständig aufgeklärt. Sicher ist aber, daß der von diesen Desodorantien bewirkte Effekt der Geruchslöschung nicht auf einer Überdeckung oder Umwandlung des Duftes beruht. Vielmehr werden Gerüche in Abhängigkeit von funktionellen Gruppen, Struktur und Größe der Moleküle gebunden und sind dann sensorisch nicht mehr wahrnehmbar. Somit sind auch die Grenzen der Wirksamkeit vorgegeben. Verzweigtkettige Kohlenwasserstoffe, mehrkernige und substituierte Aromaten, Ketone, Alkohole und Carbonsäureester werden, wie bereits oben erwähnt, nicht gebunden. Deshalb können kosmetische Produkte mit diesem Wirkstoff auch parfümiert werden.

Die bisher verwendeten Lösungsvermittler GRILLOSOL^{R} 8 C bzw. GRILLOSOL® 8 C 12 sind ausgesprochen hautfreundlich und toxikologisch unbedenklich.

Die kosmetischen Rezepturen enthalten meistens 1 bis 5 Gew.-% Zink-Ricinoleat mit Zusätzen, 1,5 bis 15 Gew.-% Lösungsvermittler sowie bis zu 90 Gew.-% Lösungsmittel aus der Gruppe der ein- und mehrwertigen Alkohole sowie gewünschtenfalls bis zu 55 Gew.-% Wasser. Derartige wasserhaltige Rezepturen neigen jedoch zur Trübung und Ausfällung einzelner Komponenten.

Für den technischen Bereich war es somit nötig, Rezepturen zu entwickeln, die auch bei höherem Wassergehalt stabil sind und welche einen preiswerten, hochwirksamen und stabilen Lösungsvermittler enthalten.

Eingehende Untersuchungen haben jetzt zu dem Ergebnis geführt, daß die hydrolysierten Enaddukte und Diels-Alder-Addukte von Ricinenfettsäuren und Maleinsäureanhydrid ausgezeichnet geeignet sind, diese Anforderungen zu erfüllen. Desodorantien, in denen die bisher verwendeten Lösungsvermittler ersetzt worden sind durch die hydrolysierten Enaddukte und Diels-Alder-Addukte von Ricinenfettsäuren und Maleinsäureanhydrid, können daher auch im technischen Maßstab verwendet werden, insbesondere zur Geruchsbeseitigung in Klaranlagen, Deponien, Haushaltungen und Krankenhäusern, in der Landwirtschaft, Tierzucht, Tierverwertung sowie in Fleisch- und Fischbetrieben.

Weiterhin wurde jetzt gefunden, daß bereits diese hydrolysierten Enaddukte und Diels-Alder-Addukte von Ricinenfettsäuren und Maleinsäureanhydrid selbst, d.h. auch ohne Zink-Ricinoleat, eine stark desodorierende Wirkung zeigen, insbesondere bei starken Ammoniak- und Schwefelwasserstoffgerüchen bzw. Emissionen dieser Stoffe.

Gegenstand der vorliegenden Erfindung sind somit zunächst Desodorantien enthaltend die hydrolysierten Enaddukte und Diels-Alder-Addukte von Ricinenfettsäuren und Maleinsäureanhydrid.

Ein weiterer Gegenstand der vorliegenden Erfindung sind Desodorantien, die außer den hydrolysierten Enaddukten und Diels-Alder-Addukten von Ricinenfettsäuren und Maleinsäureanhydrid als Komponente c) enthalten
a) Zink-Ricinoleat mit Zinkverbindungen von mehrfach hydroxylierten höheren Fettsäuren, Oxaminen und Harzsäuren,
b) Lösungmittel aus der Gruppe der Alkohole, gegebenenfalls unter Zusatz von Wasser, Triethanolamin und organischen Säuren.

Vorzugsweise enthalten die erfindungsgemäßen Desodorantien 2 bis 10 Gew.-% der Komponente a), 4 bis 30 Gew.-% der Komponente b) und 40 bis 90 Gew.-% der Komponente c).

Für die technischen Anwendungen ist es möglich, die Desodorantien bereits mit größeren Mengen Wasser zu verdünnen, so daß sie bereits mehr als 60 Gew.-% Wasser enthalten. Aber auch in diesen Rezepturen ist es vorteilhaft, wenn ein- und mehrwertige Alkohole als Lösungsvermittler, Emulgatoren und Coemulgatoren wirken. Diese Formulierungen sind dann auch bei relativ tiefen Temperaturen bis etwa 0°C stabil und flocken nicht aus, was erfahrungsgemäß die Wirksamkeit herabsetzt.

Die hydrolysierten Enaddukte und Diels-Alder-Addukte von Ricinenfettsäuren und Maleinsäureanhydrid sind neu. Sie werden nach an sich bekannten Methoden hergestellt, beispielsweise durch Umsetzung von Ricinenfettsäuren bei 120 bis 180°C mit Maleinsäureanhydrid. Sie werden anschließend mit Wasser hydrolysiert und mit einer Base neutralisiert. Vorzugsweise wird Natronlauge verwendet, es können aber auch Kalilauge, Ammoniak und/oder Triethanolamin verwendet werden. Vorzugsweise werden die Basen zunächst in geringem Überschuß zugegeben, um die Hydrolyse zu vervollständigen. Es wird dann anschließend mit einer organischen Säure wie Zitronensäure oder Milchsäure auf einen pH-Wert von 6 bis 7 eingestellt.

Weiterhin ist Gegenstand der vorliegenden Erfindung das Verfahren zur Herstellung der Desodorantien, dadurch gekennzeichnet, daß Ricinenfettsäuren bei 120 bis 180°C mit Maleinsäureanhydrid umgesetzt werden, das Umsetzungsprodukt mit Wasser hydrolysiert und mit einer Base neutralisiert wird. Gewünschtenfalls werden die so erhaltenen Desodorantien vermischt als Lösungsvermittler mit den an sich bekannten Desodorantien auf Basis von
a) Zink-Ricinoleat mit Zinkverbindungen von mehrfach hydroxylierten höheren Fettsäuren, Oxaminen und Harzsäuren und
b) Lösungsmitteln aus der Gruppe der Alkohole, gegebenenfalls unter Zusatz von Wasser, Triethanolamin und organischen Säuren.

Als Ricinenfettsäuren können erfindungsgemäß hochgereinigte Ricinenfettsäuren verwendet werden. Aus Kostengrunden können jedoch ohne weiteres die von ihren Lieferanten angebotenen technischen Ricinenfettsäuren eingesetzt werden. Diese technischen Ricinenfettsäuren enthalten im allgemeinen 75 bis 85% Ricinensäure und 1 bis 3% gesättigte Fettsäuren. Der Rest sind meistens Ölsäure und Leinölfettsäuren. Die konjugiert-doppelt ungesättigte Ricinenfettsäure ist prinzipiell in der Lage, Diels-Alder-Addukte und Enaddukte nebeneinander zu bilden. Es ist bisher nicht untersucht worden, in welchem Maße sich Enaddukte und Diels-Alder-Addukte bilden. Entscheidend ist, daß die neutralisierten Enaddukte und Diels-Alder-Addukte von Ricinenfettsäuren und Maleinsäureanhydrid untoxisch sind, keine Schleimhautreizung aufweisen, biologisch abgebaut werden können und sehr wirksame Löslichkeitsvermittler und Emulgatoren mit einer gewissen Waschwirkung sind. Sie können somit unbedenklich auch im technischen Maßstab eingesetzt werden und wirken für sich allein schon stark desodorierend.

Als Lösungsmittel werden ein- und mehrwertige Alkohole eingesetzt, insbesondere Ethanol, Isopropylalkohol, Propylenglycol, Dipropylenglycol, Glycerin und Trimethylolpropan. Diese Alkohole tragen dazu bei, daß auch verdünnte, wäßrige Lösungen nicht ausfallen und dadurch inaktiviert werden. Die verdünnteren, wäßrig-alkoholischen Lösungen können daher auch mittels Pumpen und durch Düsen appliziert werden, ohne daß es zur Verstopfung der Düsen kommt.

Die erfindungsgemäßen Desodorantien, ihre Herstellung und Anwendung sind in den nachfolgenden Beispielen näher erläutert:

### Beispiel 1

### Herstellung des hydrolysierten Enadduktes und Diels-Alder-Adduktes von Ricinenfettsäuren mit Maleinsäureanhydrid

14,5 kg technische Ricinenfettsäure (mit einem Gehalt an Ricinensäure von ca. 80%, ca 17% eines Gemisches von Ölsäure und Leinölfettsäuren und ca. 2% gesättigten Fettsäuren) werden bei 120 bis 180°C mit 16,3 kg Maleinsäureanhydrid umgesetzt. Das Gemisch wird nach dem Abkühlen mit 46 kg Wasser hydrolysiert und mit 15 kg Natronlauge neutralisiert. Anschließend wird mit 6,1 kg Triethanolamin versetzt und mit einer Zitronensaurelosung auf pH 6 bis 7 eingestellt.

### Beispiel 2

### Desodorant als Konzentrat

6,2 kg des handelsüblichen Zink-Ricinoleats mit synergistischen Zusätzen von Zinkverbindungen von mehrfach hydroxylierten höheren Fettsäuren, Oxaminen und Harzsäuren (GRILLOCIN® HY-77) werden mit 4,5 kg 1,2-Propylenglycol und 4,5 kg Propanol-2, 1,6 kg Zitronensäure und 1,6 kg Triethanolamin in 81,6 kg des gemäß Beispiel 1 hergestellten neutralisierten und hydrolysierten Produktes eingerührt und gründlich vermischt. Dieses Konzentrat kann vielseitig eingesetzt werden.

### Beispiel 3

100 bis 250 g des Konzentrates gemäß Beispiel 2 werden pro m³ Schlamm vor den Schneckenzentrifugen in Kläranlagen homogen zugesetzt. Es wird eine Geruchsabnahme bis unterhalb der Geruchsschwelle beobachtet. Mülldeponien werden mit einem wäßrigen Gemisch des Konzentrates 1:10 besprüht, bis sensorisch kein Geruch mehr wahrgenommen wird. Je nach Geruchsintensität und Geruchsart muß die Konzentration nach unten oder oben variiert werden.

### Beispiel 4

100 g Schwefel (kontaminiert mit Mercaptanen und Disulfiden) mit sehr intensivem Geruch werden mit 0,5 g des Konzentrates gemäß Beispiel 2 (mit Wasser im Verhältnis 1:1 verdünnt) oberflächlich benetzt. Die sensorische Prüfung ergab übereinstimmend, daß der desodorierte Schwefel geruchlos ist. Aus diesen Versuchen errechnet sich ein Verbrauch von etwa 2,5 kg pro t Schwefel. Unter optimierten Bedingungen kann der Verbrauch noch gesenkt werden.

### Beispiel 5

### Desodorierung des Stinkstoffes CA 460

Dieser Stinkstoff mit einem starken Maggi-artigen Geruch wird durch Zusatz von weniger als 0,1 Gew.-% des Konzentrates gemäß Beispiel 2 völlig desodoriert. Ähnlich gute Ergebnisse werden mit Zwiebelextrakt beobachtet.

### Beispiel 6

Ein Raumspray wird erhalten durch Zusammenmischen von 2 Gew.-% Zink-Ricinoleat mit Zusätzen (GRILLOCIN® HY 77), 10 Gew.-% des Konzentrates gemäß Beispiel 2, 51 Gew.-% Wasser, 25 Gew.-% Isopropylalkohol, 4 Gew.-% Isopropylmyristat, 4 Gew.-% Milchsaure und 4 Gew.-% Triethanolamin. Dieser Raumspray ist ausgezeichnet geeignet, Gerüche im Haushalt und in Krankenhäusern zu beseitigen.

Im Gegensatz zu handelsüblichen Geruchsverbesserern wie Maskomal^{R} mit starkem, esterartigen Geruch ist der erfindungsgemäße Raumspray nahezu geruchlos und bindet insbesondere Gerüche, die auf Mercaptanen und Aminen beruhen.

### Beispiel 7

Das Konzentrat gemäß Beispiel 2 wird mit Wasser im Verhältnis 1:1 bis 1:10 verdünnt. Diese Lösung kann in Reinigungswasser, Waschwasser sowie bei der Abluftreinigung über ein Wäschersystem angewendet werden. Es beseitigt in hervorragender Weise die Gerüche in der Landwirtschaft, Tierzucht, Tierverwertung sowie in Fleisch- und Fischbetrieben.

### Beispiel 8

### Rahmenrezeptur

Die im Beispiel 1 aufgeführten Natriumverbindungen der Ricinenfettsäuren und des Maleinsäureanhydrids werden 1:1 bis ca. 1:10 mit Wasser verdünnt und können dann als technisches Desodorant eingesetzt werden.

### Beispiel 9

### Richtrezeptur

5 bis 50 Gew.-% der Verbindung gemäß Beispiel 1 und 10 bis 50 Gew.-% des Alkohols (Propanol-2, Ethanol, Glycerin, Polyglycol, Ethylenglycol) werden mit Wasser auf 100% ausgeglichen; die Einstellung des pH-Wertes erfolgt mit Citronensäure, Milchsaure als Säuren bzw. Triethanolamin als Base.

### Beispiel 10

50 bis 300 g des Konzentrates aus den Beispielen 8 bzw. 9 werden pro m³ Schlamm in der Klaranlage vor der Zentrifuge homogen zugesetzt. Die übliche Geruchsschwelle wird mit diesen Desodorantien unterschritten. Besonders eignen sich die Beispiele 8 und 9 zur Desodorierung von Ammoniak und Schwefelwasserstoff.

## Patentansprüche

1. Desodorantien enthaltend die hydrolysierten Enaddukte und Diels-Alder-Addukte von Ricinenfettsäuren und Maleinsäureanhydrid.

2. Desodorantien gemäß Anspruch 1, dadurch gekennzeichnet, daß sie außer den hydrolysierten Enaddukten und Diels-Alder-Addukten von Ricinenfettsäuren und Maleinsäureanhydrid als Komponente c) enthalten
a) Zink-Ricinoleat mit Zinkverbindungen von mehrfach hydroxylierten höheren Fettsäuren, Oxaminen und Harzsäuren,
b) Lösungmittel aus der Gruppe der Alkohole, gegebenenfalls unter Zusatz von Wasser, Triethanolamin und organischen Säuren.

3. Desodorantien gemäß Anspruch 1 oder 2, dadurch gekennzeichnet, daß sie 2 bis 10 Gew.-% der Komponente a), 4 bis 30 Gew.-% der Komponente b) und 40 bis 90 Gew.-% der Komponente c) enthalten.

4. Desodorantien gemäß einem der Ansprüche 1 bis 3, dadurch gekennzeichnet, daß sie zusätzlich mehr als 60 Gew.-% Wasser enthalten.

5. Verfahren zur Herstellung von Desodorantien, dadurch gekennzeichnet, daß Ricinenfettsäuren bei 120 bis 180°C mit Maleinsäureanhydrid umgesetzt werden, das Umsetzungsprodukt mit Wasser hydrolysiert und mit einer Base neutralisiert wird, woraufhin gewünschtenfalls die so erhaltenen Desodorantien vermischt werden als Lösungsvermittler mit den an sich bekannten Desodorantien auf Basis von
a) Zink-Ricinoleat mit Zinkverbindungen von mehrfach hydroxylierten höheren Fettsäuren, Oxaminen und Harzsäuren und
b) Lösungmitteln aus der Gruppe der Alkohole, gegebenenfalls unter Zusatz von Wasser, Triethanolamin und organischen Säuren

6. Verwendung von Desodorantien gemäß einem der Ansprüche 1 bis 4 zur Geruchsbeseitigung in Kläranlagen, Deponien, Haushaltungen und Krankenhäusern, in der Landwirtschaft,Tierzucht, Tierverwertung sowie in Fleisch- und Fischbetrieben.

## Claims

1. Deodorants containing the hydrolyzed ene-adducts and Diels-Alder adducts of ricinene fatty acids and maleic anhydride.

2. Deodorants according to claim 1, characterized in that they contain
a) zinc ricinoleate with zinc compounds of polyhydroxylated higher fatty acids, oxamines and resin acids,
b) solvents taken from the group of alcohols, optionally with the addition of water, triethanolamine and organic acids,
in addition to the component
c) comprising the hydrolyzed ene-adducts and Diels-Alder adducts of ricinene fatty acids and maleic anhydride.

3. Deodorants according to claim 1 or 2, characterized in that they contain
from 2 to 10% by weight of the component a),
from 4 to 30% by weight of the component b) and
from 40 to 90% by weight of the component c).

4. Deodorants according to anyone of claims 1 to 3, characterized in that they additionally contain more than 60% by weight of water.

5. A process for preparing deodorants, characterized in that ricinene fatty acids are reacted with maleic anhydride at from 120 °C to 180 °C, the reaction product is hydrolyzed with water and neutralized with a base, whereupon, if desired, the deodorants thus obtained are mixed as solubilizers with the per se known deodorants based on
a) zinc ricinoleate with zinc compounds of polyhydroxylated higher fatty acids, oxamines and resin acids and
b) solvents taken from the group of alcohols, optionally with the addition of water, triethanolamine and organic acids.

6. Use of deodorants according to anyone of claims 1 to 4 for the deodorization in sewage treatment plants, disposal areas, household and hospital use, in agriculture, animal breeding, animal utilization and in meat and fish processing plants.

## Revendications

1. Désodorants contenant les "p.a.-ène" hydrolysés et les produits d'addition de Diels-Alder d'acides gras de ricin et d'anhydride maléique.

2. Désodorants selon la revendication 1, caractérisés en ce qu'ils contiennent, en plus des "p.a.-ène" hydrolysés et des produits d'addition de Diels-Alder d'acides gras de ricin et d'anhydride maléique comme composant c) :
a) du ricinoléate de zinc avec des composés de zinc d'acides gras supérieurs polyhydroxylés, d'oxamines et d'acides résiniques,
b) un solvant choisi dans le groupe formé des alcools, éventuellement avec addition d'eau, de triéthanolamine et d'acides organiques.

3. Désodorants selon la revendication 1 ou 2, caractérisés en ce qu'ils contiennent 2 à 10 % en poids de composant a), 4 à 30 % en poids de composant b) et 40 à 90 % en poids de composant c).

4. Désodorants selon une des revendications 1 à 3, caractérisés en ce qu'ils contiennent en outre plus de 60 % en poids d'eau.

5. Procédé de préparation de désodorants, caractérisé en ce qu'on fait réagir des acides gras de ricin, à une température comprise entre 120 et 180°C, avec de l'anhydride maléique, on hydrolyse le produit de réaction avec de l'eau et on le neutralise avec une base, après quoi, si on le souhaite, on mélange les désodorants ainsi obtenus, comme agents de solubilisation, avec les désodorants connus à base de :
a) ricinoléate de zinc avec des composés de zinc d'acides gras supérieurs polyhydroxylés, d'oxamines et d'acides résiniques,
b) solvant choisi dans le groupe formé des alcools, éventuellement avec addition d'eau, de triéthanolamine et d'acides organiques.

6. Utilisation des désodorants selon une des revendications 1 à 4 pour éliminer les odeurs dans des stations d'épuration, des décharges de déchets, les ménages et les hôpitaux, les exploitations agricoles, les élevages, l'industrie de transformation des animaux, ainsi que dans les boucheries et les poissonneries.
